(19) **Europäisches Patentamt**
**European Patent Office**
**Office européen des brevets**

(11) **EP 2 902 024 B1**

(12) **EUROPEAN PATENT SPECIFICATION**

(45) Date of publication and mention
of the grant of the patent:
**29.11.2017   Bulletin 2017/48**

(51) Int Cl.:
**A61K 31/4184** *(2006.01)*   **A61K 31/426** *(2006.01)*
**A61P 33/04** *(2006.01)*   **A61P 33/10** *(2006.01)*

(21) Application number: **12885371.0**

(86) International application number:
**PCT/IB2012/055166**

(22) Date of filing: **27.09.2012**

(87) International publication number:
**WO 2014/049397 (03.04.2014 Gazette 2014/14)**

(54) **SYNERGIC COMPOSITION OF NITAZOXANIDE AND MEBENDAZOLE, METHODS FOR THE PREPARATION THEREOF, AND USE OF SAID COMPOSITION FOR THE TREATMENT OF HUMAN PARASITOSIS**

SYNERGISTISCHE ZUSAMMENSETZUNG AUS NITAZOXANID UND MEBENDAZOL, VERFAHREN ZUR HERSTELLUNG DAVON UND VERWENDUNG DIESER ZUSAMMENSETZUNG ZUR BEHANDLUNG VON MENSCHLICHEN PARASITOSEN

COMPOSITION SYNERGIQUE DE NITAZOXANIDE ET DE MÉBENDAZOLE, PROCÉDÉS DE PRÉPARATION CORRESPONDANTS ET UTILISATION DE CETTE COMPOSITION DANS LE TRAITEMENT DE LA PARASITOSE HUMAINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**05.08.2015   Bulletin 2015/32**

(73) Proprietor: **Siegfried Rhein S.A. De C.V.**
**Álvaro Obregón, C.P. 01210 México, D.F. (MX)'**
**(MX)**

(72) Inventor: **FIORE, Esteban Alejandro**
**Ciudad de Buenos Aires**
**1417 (AR)**

(74) Representative: **Eder, Michael**
**df-mp Dörries Frank-Molnia & Pohlman**
**Patentanwälte Rechtsanwälte PartG mbB**
**Theatinerstrasse 16**
**80333 München (DE)**

(56) References cited:
- **F PALOMARES-ALONSO ET AL.: 'Efficacy of nitazoxanide, tizoxanide and tizoxanide/albendazol sulphoxide combination against Taenia crassiceps cysts' JOURNAL OF ANTIMICROBIAL CHEMOTHERAPY vol. 59, 2007, pages 212 - 218, XP055245216**
- **R RODRIGUEZ-GARCIA ET AL. REVISTA OF GASTROENTEROLOGIA MEXICANA 0375-0906 vol. 64, no. 3, 1999, pages 122 - 126, XP055249756**
- **C E DAVILA-GUTIERREZ ET AL.: 'Nitazoxanide compared with quinfamide and mebendazol in the treatment of helminthic infections and intestinal protozoa in children' AMERICAN JOURNAL OF TROPICAL MEDICINE AND HYGIENE vol. 66, no. 3, 2002, pages 251 - 254, XP055245221**

Description

## TECHNICAL FIELD OF THE INVENTION

This invention relates to a synergic combination of nitazoxanide and mebendazole for the treatment of human parasitosis caused by protozoa and helminths.

This association combines the nitazoxanide and mebendazole compounds, showing an unexpected synergism, widening the spectrum and enhancing the antihelmintic and antiprotozoal action of both active ingredients.

## BACKGROUNDS OF THE INVENTION

Intestinal parasitic infections are among the most significant causes of morbidity and mortality, particularly in developing countries. Helminth infections are a public health problem worldwide. For example, helminthiasis affect chronically about one third of the world population, with an estimated one million cases of geothelminths, 900 million prevalent cases of trichuriasis, and 500 million cases of anclyostoma.

Parasitic infections affect mainly children of school age and are often transmitted where hygiene/sanitation are poor. This child population affected by intestinal parasites is due to their immunological immaturity and the poor development of hygiene. These parasitosis can lead to negative consequences, both physical as from the cognitive point of view, in many parasitized children.

Most parasites are transmitted by fecal-oral route, particularly by ingestion of water and/or food contaminated with the various infectious forms of the parasites. Geohelminths require a maturation process in the soil to infect another host and can do so through larvae that penetrates the skin.

A feature of the incidence of parasites in school children is the high incidence of infection of more than one species. In an epidemiological study conducted in the province of Mendoza, Argentina, an overall prevalence of intestinal parasites of 80,5% was observed, with values ranging from 88% (age group 5-10 years) and 63,8 % (age group of 11-14 years), where 37,6% of positive presented a single species, while in the rest parasitic associations of up to 4 different genera were found [Salomón, M.C. et al. Parasitol. Latinoam. V.62 n.1-2 Santiago, Jun 2007].

An ideal parasite is one that proves to have a wide range to cover as many intestinal parasites (helminths and protozoa) as possible, easy delivery scheme; good biosafety profile in both children and adults and also that in the cost-benefit analysis justifies its use in the population scope.

There have been various studies regarding the associations of different antiparasitics, as in the case of a clinical trial conducted with the association of albendazole with praziquantel against trichuris-trichuria, where no synergic effect was observed for both drugs [Sirivichayakul, C. et al., Southeast Asian J. Trop. Med. Public Health. 2001 32: 297-301]. Neither synergism was observed in the treatment of geothelmiths and schistosomiasis in school children with the albendazole and praziquantel combination [Olds, G.R. et al, 1999, J. Infect. Diseases 179: 996-1003), as is described in a study where albendazole (500mg) and mebendazole (400mg) were administrable as a single dose for the treatment of ancylostoma and other helminths and in 200 infected children [Soukhathammavong, PA. Et. al, PLoS Negl Trop Dis. 2012 Jan;6(1):e1417. Epub 2012 Jan 3], where no synergic effect was observed in the cure rate against ancylostoma. In another epidemiological study performed in northeastern Argentina, it was found that 74% of the children were poly parasitized. The most frequent combination found was Enterobius vermicularis, Blastocystis hominis and/or Giardia [Milano, A. et al., Enteroparasitosis infantil intestinal en Argentina. Medicina (Buenos Aires) 2000;60: 23-4].

The compound **nitazoxanide** was disclosed as a product in the United States Patent US 3 950 351 and its equivalents, whose owner is **S.P.R.L. Phavic** and the priority date is **8-8-73.** Then, the patent US 5 387 598 owned by **Romark** and with priority date **04-13-94** describes a formulation containing Nitazoxanide and Tizoxanide.

*In vitro* efficacy of nitazoxanide, tizoxanide and tizoxanide/albendazole sulphoxide were tested against Taenia crassiceps cysts byPalomares-Alonso et al. [J Antimicrob Chemother 2007, 59 (2): 212-218.].

The **Mebendazole** compound was described as a product in the United State patent US 3 657 267 and their equivalents, owned by **Janssen Pharmaceutica N.V.** and which priority date is **06-20-69.**

Due to the resistance growing development to antiparasitics by the intestinal nematodes, it was found that it was necessary to investigate new alternative strategies to the pharmacological control of parasitosis diseases. Combining different antiparasitics was considered a strategy to reach a wider action spectrum and enhance the anthelmintic and antiprotozoal action of both active ingredients.

## BRIEF DESCRIPTION OF THE FIGURE

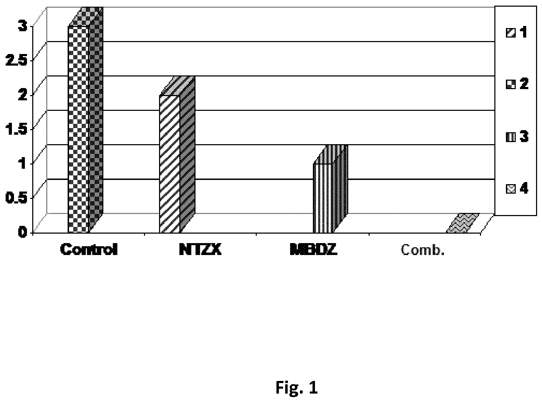

Figure 1 illustrates the evaluation results of L4 helminth larvae.

## DETAILED DESCRIPTION OF THE INVENTION

By associating Mebendazole (MBDZ) with Nitazoxanide (NTZX), it was surprisingly achieved to significantly broaden the spectrum of MBDZ against protozoa and strengthen NTZX anthelmintic action. In addition, the systemic action of the active metabolite of the NTZX, tizoxanide, and the treatment of some systemic forms of parasitosis are maintained by using the pharmaceutical combination of MBDZ and NTZX. Further, the synergic effects of the pharmaceutical combination of MBDZ and NTZX achieve a wider antiparasitic spectrum, while maintaining effectiveness and safety profiles of both active ingredients separately. All these effects allow this invention to differ from other typical antiparasitic treatments with narrower spectra. The synergic effects of the pharmaceutical combination of MBDZ and NTZX are, therefore, the main objective of this invention.

Therefore, this invention generally relates to a synergic pharmaceutical combination comprising a therapeutically effective amount of Nitazoxanide antiparasitic and a therapeutically effective amount of Mebendazole antiparasitic for use in the treatment of human parasitosis.

In one aspect, the invention relates to a pharmaceutical composition adapted to be orally administrable as antiparasitic comprising the combination of a therapeutically effective amount of Nitazoxanide antiparasitic with a therapeutically effective amount of Mebendazole antiparasitic, along with pharmaceutically acceptable excipients.

In some embodiments such pharmaceutical composition for oral delivery may be a coated tablet.

In some embodiments such pharmaceutical composition for oral delivery may be a powder for extemporaneous reconstitution.

Furthermore, in certain embodiments such pharmaceutical composition for oral delivery may be administrable once or twice a day.

The therapeutically effective doses of Nitazoxanide used in the oral delivery pharmaceutical composition of the invention may be comprised within the range of 50 mg to 1200 mg and the therapeutically effective doses of mebendazole in the range of 20 to 500 mg, preferably containing, per adult dosage unit, 500 mg of nitazoxanide and 100 mg of Mebendazole. The powder for extemporaneous reconstitution used for the pediatric formulation preferably contains 100 mg of nitazoxanide and 50 mg Mebendazole. Finally, the oral delivery pharmaceutical composition of the invention which is administered once a day preferably comprises 1000 mg of Nitazoxanide and 200 mg of Mebendazole.

In a further aspect, this invention further relates to processes for preparing the pharmaceutical composition for oral delivery which comprises granulating, mixing and tableting therapeutically effective amounts of the active ingredients nitazoxanide and mebendazole, along with pharmaceutically acceptable excipients and optionally coating the tablets obtained.

In another aspect, this invention relates to processes for preparing the pharmaceutical composition of pediatric oral delivery which comprises the mixture of therapeutically effective amounts of the active ingredients with Nitazoxanide and Mebendazole along with pharmaceutically acceptable excipients. The powder for extemporaneous reconstitution is thus obtained.

A further object of this invention relates to the Nitazoxanide antiparasitic along with the Mebendazole antiparasitic for use in the treatment of human parasitosis, particularly for use in the treatment of human parasitosis caused by protozoa and helminths.

The synergism has been demonstrated by *in vitro* studies showing the anthelmintic and antiprotozoal action of the pharmaceutical combination of the present invention according to that described in **example 4.**

## WORKING EXAMPLES

### EXAMPLE 1

The process for preparing a formulation of coated tablets for delivery every 12 hours is described.

For coated tablet

**CORE**

|                     | mg     | %     |
|---------------------|--------|-------|
| Nitazoxanide        | 500,00 | 53,76 |
| Mebendazole         | 100,00 | 10,75 |
| Corn starch         | 40,00  | 4,30  |
| Pregelatinized starch | 70,00 | 7,53 |
| Povidone K30        | 40,00  | 4,30  |

(continued)

|  | mg | % |
| --- | --- | --- |
| Microcrystalline cellulose | 91,00 | 9,79 |
| Sodium glycolate starch | 43,00 | 4,62 |
| Talc | 8,00 | 0,86 |
| Magnesium stearate | 8,00 | 0,86 |
| Purified water | Approx. 182 | |

**COATING**

|  | mg | % |
| --- | --- | --- |
| Hydroxypropylmethylcellulose | 17,00 | 1,83 |
| Titanium dioxide | 9,00 | 0,97 |
| Triacetine | 3,50 | 0,38 |
| Yellow iron oxide | 0,50 | 0,05 |
| Purified water | Approx. 220 | |

## METHOD OF PREPARATION

1. Solve Povidone K30 in purified water.
2. Sieve through a 1 mm mesh and transfer to the mixer:

nitazoxanide, nebendazole, corn starch and pregelatinized starch.

3. Mix for 2 minutes and add the solution obtained in item 1. Amass until the point of granulation is obtained.
4. Calibrate the granulate through a 3 mm mesh.
5. Dry the calibrated granulation until reaching a 2 to 4% moisture.
6. Calibrate the granulate through a 1 mm mesh.
7. Mix the calibrated granulate with the microcrystalline cellulose, the sodium glycolate starch, the talc and the magnesium stearate.
8. Tablet to 900 mg $\pm$ 5% mg of theoretical weight.
9. Prepare the coating by perfectly suspending the yellow iron oxide and the titanium dioxide in the water and subsequently adding hydroxypropylmethylcellulose and triacetine.
10. Coat the tablets until a theoretical weight of about 930 mg $\pm$ 5% is achieved.

## EXAMPLE 2

The details of the preparation process of the powder for oral suspension for pediatric use deliverable every 12 hours are shown below.

34 g of powder for obtaining 100 ml of reconstituted suspension contain

|  | g | % |
| --- | --- | --- |
| Nitazoxanide | 2,000 | 5,882 |
| Mebendazole | 1,000 | 2,941 |
| Sodium benzoate | 0,200 | 0,588 |
| Refined sugar | 29,599 | 87,056 |

(continued)

|  | g | % |
|---|---|---|
| Xanthan Gum | 0,800 | 2,353 |
| Anhydrous citric acid | 0,200 | 0,588 |
| Dihydrate sodium citrate | 0,050 | 0,147 |
| FD & C Red 40 dye | 0,001 | 0,003 |
| Essence of strawberry powder | 0,150 | 0,441 |
|  | 34,000 | 100,00 |

## METHOD OF PREPARATION

1. Grind the sugar to fine powder, set aside 10% of the grinding, put the rest in a suitable mixer.

2. Mix the FD & C red 40 dye, the powdered strawberry Essence and the xanthan gum, grind the mixture to a fine powder. Add it to the mixer of item 1. Mix for 5 minutes.

3. Add the previously milled to a fine powder nitazoxanide, mebendazole, sodium benzoate, dihydrate sodium citrate, anhydrous citric acid to the mix of item 1. Use the set aside 10% of sugar to rinse the equipment where the grindings were performed and add it to the mixer. Mix for 15 minutes.

4. Pack 34 g per flask.

5. Prior to use, reconstitute with 100 ml of drinking water.

## EXAMPLE 3

The process for preparing a formulation of coated tablets deliverable every 24 hours is described.
Per coated tablet

**CORE**

|  | mg | % |
|---|---|---|
| Nitazoxanide | 1000,00 | 56,82 |
| Mebendazole | 200,00 | 11,36 |
| Corn starch | 70,00 | 3,98 |
| Pregelatinized starch | 130,00 | 7,39 |
| Povidone k30 | 75,00 | 4,26 |
| Microcrystalline cellulose | 120,00 | 6,82 |
| Sodium glycolate starch | 75,00 | 4,26 |
| Talc | 15,00 | 0,85 |
| Magnesium stearate | 15,00 | 0,85 |
| Purified water | Aprox. 363 |  |

**COATING**

|  | mg | % |
|---|---|---|
| Hydroxypropylmethylcellulose | 34,00 | 1,93 |
| Titanium dioxide | 18,00 | 1,02 |
| Triacetine | 7,00 | 0,40 |
| Yellow iron oxide | 1,00 | 0,06 |
| Purified water | Aprox. 440 | |

## METHOD OF PREPARATION

1. Solve povidone K30 in purified water.
2. Sieve through a 1 mm mesh and transfer to the mixer:
3. Nitazoxanide, mebendazole, corn starch and pregelatinized starch
4. Mix for 2 minutes and add the solution obtained in item 1. Amass until the granulation point obtention.
5. Calibrate the granulate through a 3 mm mesh.
6. Dry the calibrated granulate until a 2 to 4% moisture is obtained.
7. Calibrate the dry granulate through a 1 mm mesh.
8. Mix the calibrated granulate with the microcrystalline cellulose, the sodium glycolate starch, the talc and the magnesium stearate.
9. Tablet to 1.700 mg $\pm$ 5% mg of theoretical weight.
10. Prepare the coating by perfectly suspending the yellow iron oxide and titanium dioxide in the water and subsequently adding hydroxypropylmethylcellulose and triacetine.
11. Coat the tablets until obtaining a theoretical weight of 1760 mg $\pm$ 5%.

## EXAMPLE 4

### *IN VITRO* TESTS

*In vitro* tests were performed to assess and know the susceptibility and resistance of various parasites to the pharmaceutical combination of MBDZ and NTZX.

Methods:

### A) Anthelmintic *in vitro* determination:

1) In order to determine the *in vitro* anthelmintic effectiveness of the pharmaceutical combination of NTZX and MBDZ the "inhibition of larval migration test" was used, which was developed by Wagland et al. (1992) and modified by Rable et al. (1994) and Paolini et al. 2004). The objective of this study was to use the inhibition of larval migration test to study the anthelmintic effect of the two active ingredients separately and in combination, in the migration of L4 (larva or adult worm) of different kinds of helminths (eg. trichuris spp), in doses of 100 $\mu$g/ml (100 $\mu$g/ml NTZX, 100 $\mu$g/ml MBDZ and 100 $\mu$g/ml of the combination [50 $\mu$g/ml NTZX and 50 $\mu$g/ml MBDZ]).
The rats of the Sprague-Dawley vivarium strain, parasites naive, were infected with different kinds of helminths (eg. Trichuria trichuris infected eggs). Approximately 3 g of feces were placed in culture bottles. The feces were cultured to produce worms or larvae (L4 stage) at a temperature of 37°C for 72 hours. In order to determine the *in vitro* anthelmintic effectiveness the larval migration inhibition test was used. For such test, 15 mm long by 10 mm diameter filters with transparent acrylic tubes were built, to which a nylon mesh of 20 microns was adhered on one end. 2 ml plastic tubes were used, to which the subject active ingredients (100 $\mu$g/ml NTZX, 100 $\mu$g/ml MBDZ and 100 mg/ml of the combination of NTZX and MBDZ and a control with no active drugs) were added, diluted in 0,4 ml and 100 to 200 L4 and suspended in 0,1 ml of aqueous solution. The tubes were incubated at 37°C for 16 hours. The total content of these tubes, 0,5 ml, was transferred to the filters located within the acrylic tubes and grown at room temperature for 18 hours to allow the larvae to migrate through the filters within the chamber. Then, the filters were removed from the chambers and the total number of larvae in the filters and in chambers was counted.
The larval migration inhibition (LMI) was determined using the following formula:

$$LMI = \frac{A-B}{A} \times 100$$

Wherein A= larvae migrated ratio in the control and B= larvae migrated ratio in the treatments.
The larval migration inhibition test results obtained were as follows:

- **Control Group:** there was a 100% parasites migration, therefore the **LMI = 0.**
- **Group treated with NTZX:** there was a 40% migration, therefore the **LMI = 60%.**
- **Group treated with MBDZ:** there was a 30% migration, therefor the **LMI =70%.**
- **Group treated with the combination (NTZX+MBDZ):** there was a 10% migration, therefore the **LMI=90%.**

2) Another method for the larval motility assessment was using the "scale of larval motility" in the various tubes with the different drugs and the control group. The scale of motility is a scale from 0 to 3 points, wherein 0 = death; 1 = very low motility; 2 = low motility and 3 = normal motility.

The following are the results obtained by this method:

1) **Control culture,** with no active drug: complete parasite viability at 72 hours. Motility scale = 3
2) **Culture with NTZX:** Motility Scale = 2 in all observed parasites.
3) **Culture with MBDZ:** Motility Scale = 1 in all observed parasites.
4) **Culture with the NTZX+MBDZ combination** = 0 in all observed parasites.

Based on the above assessed inhibition test, it is shown that the combination of the drugs NTZX and MBDZ has a greater synergic effect on helminthes L4 than that observed with the two active ingredients NTZX and MBDZ separately.
Figure 1 illustrates the results of the L4 helminth larvae assessment, wherein: control = 3; NTZX = 2; MBDZ = 1; Combination (NTZX + MBDZ) = 0. Motility scale: 0 = death; 1 = very low motility; 2 = low motility; 3 = normal motility.

**B) Antiprotozoal *in vitro* determination:**

1) The *in vitro* antiprotozoal effectiveness of the pharmaceutical combination of NTZX with MBCZ was determined in an intestinal Giardia culture as compared with the NTZX and MBDZ active ingredients separately. In this study the morphology, the adhesion and the viability of the Giardias trophozoites was assessed in *in vitro* cultures. Materials and method: The antiparasitic agents used were NTZX at a 2 μg/ml concentration; MBDZ at a 2 μg/ml concentration and a pharmaceutical combination of 1 μg/ml NTZX and 1 μg/ml MBDZ. The trophozoites were obtained from the intestines of rats (Sprague-Dawley vivarium strain) previously infected with ***Giardia intestinalis.***

The trophozoites were isolated in a BI-S33 culture media containing 10% bovine serum without added antibiotic. From this media, 4,5ml were extracted, which were separated in 8 glass tubes with screw caps. These tubes (labeled with the active ingredients separately, the combination and other as control) were inoculated with the corresponding active ingredients, leaving the control drug naive. 4 of the tubes were exposed to the drugs for 4 hours and the other 4 tubes were exposed to the drugs for 24 hours. After the established periods of time were over (4 to 24 hours) the tubes underwent centrifugation (5 minutes at 500 rpm), where the supernatant was extracted and removed. The decanted material was then dyed with a 0,1% eosin solution to assess the viability of trophozoites:
a. The adhesion and growth uniformity of trophozoites was assessed, using an optical microscope in 5-10 fields (x100 and x200). For this, a table was drafted describing the following:

| Ranking | Number of adhered trophozoites |
| --- | --- |
| 0 | None |

(continued)

| Ranking | Number of adhered trophozoites |
|---------|-------------------------------|
| 1 | <1 |
| 2 | 1-10 |
| 3 | 10-25 |
| 4 | 25-50 |
| 5 | 50-100 |
| 6 | 100-250 |
| 7 | 250-500 |
| 8 | 500-1000 |
| 9 | 1000-2000 |
| 10 | clusters |

b. The trophozoites viability rate was assessed, by using a Neubauer camera to the optical microscope. The percentage of non-viable trophozoites was estimated as compared with the total state of trophozoites (live and immobile).
c. The mobility and morphology of the trophozoites was assessed. Mobility is divided into:

| Ranking | Mobility |
|---------|----------|
| 3 | Progressive, fast or slow |
| 2 | Non progressive or in situ |
| 1 | immobile |

The normal morphology of trophozoites under the optical microscope is characterized by the following: unicellular organism, with pyriform morphology, bilateral symmetry, flagellated, bi-nucleated (vacuolar complex) on its dorsal face. Any change in morphology was correspondingly detailed.

Results:

Based on the microscopic morphological changes following exposure to the different active ingredients, the following was found:

- It was observed that the morphological changes were correlated with loss of viability thereof in the group treated with the combination of both active ingredients after 24 hours of exposure. The main changes were: total loss of their pyriform shape, where many folded trophozoites were observed; loss of the bilateral symmetry and of the dorsal vacuolar complex; almost total immobility of the trophozoites. Adhered trophozoites were observed in a covered or almost completely covered field, forming nests or flaps.

- In cases where they were exposed to NTZX for 24 hours, globular trophozoites (very enlarged), a moderate amount of folded trophozoites and partial loss of dorsal vacuolar complex were observed. A low number of motile trophozoites (mostly in situ) and erratic movements were observed. The amount of adhered trophozoites was moderate.

- In cases of MBDZ exposure for 24 hours, it was observed that some trophozoites were globular, some folded, some of distorted forms (partial loss of bilateral symmetry).

- In the control group, no changes were observed in the pyriform shape nor in the progressive motility of the trophozoites, same maintaining the flagella and the dorsal vacuolar complex.

| Hours of drug expo-sure | Drugs | Non viable Tropho-zoites % | Ranking of tropho-zoites adhesion | Viability rate (%) | Mobili-ty Ranking | Morpho-logical changes |
|---|---|---|---|---|---|---|
| 4 hours | Control | 0 | 0 | 100 | 3 | No changes |
| | DZ | 30 | 4 | 70 | 2 | |
| | NTZX | 43,5 | 5 | 56,5 | 2 | |
| | MBDZ + NTZX | 66,5 | 7 | 33,5 | 1 | |
| 24 hours | Control | 0 | 0 | 100 | 3 | No changes |
| | MBDZ | 48 | 5 | 52 | 1 | |
| | NTZX | 67,5 | 7 | 32,5 | 1 | |
| | MBDZ + NTZX | 93,7 | 10 | 6,3 | 1 | |

The above *in vitro* tests show the antiparasitic superiority, in helminth and protozoan, in both, the active ingredients separately (MBDZ and NTZX) and in the pharmaceutical combination of MBDZ with NTZX. Also, these tests show a synergic effect of the pharmaceutical combination of MBDZ with NTZX on the two active ingredients MBDZ and NTZX separately. The antigiardial effect of MBDZ is enhanced by the combination with NTZX, and the same happens with the antiprotozoal effect of NTZX when it is combined with MBDZ.

**Claims**

1. A synergic pharmaceutical composition adapted to be orally administrable as antiparasitic comprising the combination of a therapeutically effective amount of the nitazoxanide antiparasitic with a therapeutically effective amount of mebendazole antiparasitic, along with pharmaceutically acceptable excipients.

2. A pharmaceutical composition adapted to be orally administrable according to claim 1, wherein the pharmaceutical composition is a coated tablet.

3. A pharmaceutical composition adapted to be orally administrable according to claim 1, wherein the pharmaceutical composition is a powder for extemporaneous reconstitution.

4. A pharmaceutical composition adapted to be orally administrable according claims 1 to 3, wherein the therapeutically effective amount of nitazoxanide ranges between 50 mg and 1200 mg and the therapeutically effective amount of mebendazole ranges between 20 mg and 500 mg.

5. A pharmaceutical composition adapted to be orally administrable according to claim 4, wherein the therapeutically effective amount of nitazoxanide is 500 mg per dosage unit and the therapeutically effective amount of mebendazole is 100 mg per dosage unit.

6. A pharmaceutical composition adapted to be orally administrable according to claim 4, wherein said pharmaceutical composition is for pediatric use and wherein the therapeutically effective amount of nitazoxanide is 100 mg per dosage unit and the therapeutically effective amount of mebendazole is 50 mg per dosage unit.

7. A pharmaceutical composition adapted to be orally administrable according to claim 4, wherein the pharmaceutical composition is further adapted to be administrable every 12 hours.

8. A pharmaceutical composition adapted to be orally administrable according to claim 4, wherein the pharmaceutical composition is further adapted to be administrable once a day.

9. A pharmaceutical composition adapted to be orally administrable according to claim 8, wherein the therapeutically effective amount of nitazoxanide is 1000 mg and the therapeutically effective amount of mebendazole is 200 mg per dosage unit.

10. A pharmaceutical combination comprising a therapeutically effective amount of nitazoxanide antiparasitic and a therapeutically effective amount of mebendazole antiparasitic according to claim 1 for the treatment of human parasitosis.

11. A process for preparing a pharmaceutical composition adapted to be orally administrable according to claim 1, comprising granulating, mixing and tableting a therapeutically effective amount of nitazoxanide antiparasitic with a therapeutically effective amount of mebendazole antiparasitic along with pharmaceutically acceptable excipients.

12. A process for preparing the pharmaceutical composition adapted to be orally administrable according to claim 11, further comprising coating the obtained pharmaceutical composition.

13. A process for preparing a pharmaceutical composition of extemporaneous reconstitution adapted to be orally administrable according to claim 3, comprising mixing a therapeutically effective amount of nitazoxanide antiparasitic with a therapeutically effective amount of mebendazole antiparasitic, along with pharmaceutically acceptable excipients.

14. A nitazoxanide antiparasitic in combination with a mebendazole antiparasitic for use in the treatment of human parasitosis.

15. The combination for use according to claim 14, wherein the human parasitosis is caused by protozoa and helminths.

**Patentansprüche**

1. Synergistische pharmazeutische Zusammensetzung angepasst zur oralen Verabreichung als Antiparasitikum, umfassend die Kombination aus einer therapeutisch wirksamen Menge des Antiparasitikums Nitazoxanid mit einer therapeutisch wirksamen Menge des Antiparasitikums Mebendazol zusammen mit pharmazeutisch annehmbaren Hilfsstoffen.

2. Pharmazeutische Zusammensetzung angepasst zur oralen Verabreichung nach Anspruch 1, wobei die pharmazeutische Zusammensetzung eine beschichtete Tablette ist.

3. Pharmazeutische Zusammensetzung angepasst zur oralen Verabreichung nach Anspruch 1, wobei die pharmazeutische Zusammensetzung ein Pulver zur Rekonstitution nach Rezept ist.

4. Pharmazeutische Zusammensetzung angepasst zur oralen Verabreichung nach einem der Ansprüche 1 bis 3, wobei die therapeutisch wirksame Menge an Nitazoxanid im Bereich zwischen 50 mg und 1200 mg liegt und die therapeutisch wirksame Menge an Mebendazol im Bereich zwischen 20 mg und 500 mg liegt.

5. Pharmazeutische Zusammensetzung angepasst zur oralen Verabreichung nach Anspruch 4, wobei die therapeutisch wirksame Menge an Nitazoxanid 500 mg pro Dosierungseinheit beträgt und die therapeutisch wirksame Menge an Mebendazol 100 mg pro Dosierungseinheit beträgt.

6. Pharmazeutische Zusammensetzung angepasst zur oralen Verabreichung nach Anspruch 4, wobei die pharmazeutische Zusammensetzung zur pädiatrischen Verwendung ist, und wobei die therapeutisch wirksame Menge an Nitazoxanid 100 mg pro Dosierungseinheit beträgt und die therapeutisch wirksame Menge an Mebendazol 50 mg pro Dosierungseinheit beträgt.

7. Pharmazeutische Zusammensetzung angepasst zur oralen Verabreichung nach Anspruch 4, wobei die pharmazeutische Zusammensetzung ferner dafür ausgelegt ist, alle 12 Stunden verabreicht zu werden.

8. Pharmazeutische Zusammensetzung angepasst zur oralen Verabreichung nach Anspruch 4, wobei die pharmazeutische ferner dafür ausgelegt ist, einmal pro Tag verabreicht zu werden.

9. Pharmazeutische Zusammensetzung angepasst zur oralen Verabreichung nach Anspruch 8, wobei pro Dosierungseinheit die therapeutisch wirksame Menge an Nitazoxanid 1000 mg beträgt und die therapeutisch wirksame Menge an Mebendazol 200 mg beträgt.

10. Pharmazeutische Zusammensetzung nach Anspruch 1, eine therapeutisch wirksame Menge an dem Antiparasitikum Nitazoxanid und eine therapeutisch wirksame Menge an dem Antiparasitikum Mebendazol umfassend, zur Behandlung von Parasitose beim Menschen.

11. Verfahren zur Herstellung einer pharmazeutischen Zusammensetzung angepasst zur oralen Verabreichung nach Anspruch 1, das Granulieren, das Mischen und Tablettieren einer therapeutisch wirksamen Menge des Antiparasitikums Nitazoxanid mit einer therapeutisch wirksamen Menge des Antiparasitikums Mebendazol zusammen mit pharmazeutisch annehmbaren Hilfsstoffen umfassend.

12. Verfahren zur Herstellung einer pharmazeutischen Zusammensetzung angepasst zur oralen Verabreichung nach Anspruch 11, ferner das Beschichten der erhaltenen pharmazeutischen Zusammensetzung umfassend.

13. Verfahren zur Herstellung einer pharmazeutischen Zusammensetzung angepasst zur oralen Verabreichung nach Anspruch 3, umfassend das Mischen einer therapeutisch wirksamen Menge des Antiparasitikums Nitazoxanid mit einer therapeutisch wirksamen Menge des Antiparasitikums Mebendazol zusammen mit pharmazeutisch annehmbaren Hilfsstoffen.

14. Nitazoxanid als Antiparasitikum in Kombination mit dem Antiparasitikum Mebendazol zur Verwendung bei der Behandlung von Parasitose beim Menschen.

15. Kombination zur Verwendung nach Anspruch 14, wobei die Parasitose beim Menschen durch Protozoen und Helminthe verursacht ist.

**Revendications**

1. Composition pharmaceutique synergique apte à être administrée par voie orale comme antiparasite, comprenant la combinaison d'une quantité efficace sur le plan thérapeutique de l'antiparasite nitazoxanide avec une quantité efficace sur le plan thérapeutique de l'antiparasite mébendazole, ainsi que des excipients acceptables sur le plan pharmaceutique.

2. Composition pharmaceutique apte à être administrée par voie orale selon la revendication 1, dans laquelle la composition pharmaceutique est un comprimé pelliculé.

3. Composition pharmaceutique apte à être administrée par voie orale selon la revendication 1, dans laquelle la composition pharmaceutique est une poudre pour reconstitution extemporanée.

4. Composition pharmaceutique apte à être administrée par voie orale selon les revendications 1 à 3, dans laquelle la quantité efficace sur le plan thérapeutique du nitazoxanide se trouve entre 50 mg et 1200 mg et la quantité efficace sur le plan thérapeutique du mébendazole se trouve entre 20 mg et 500 mg.

5. Composition pharmaceutique apte à être administrée par voie orale selon la revendication 4, dans laquelle la quantité efficace sur le plan thérapeutique du nitazoxanide est de 500 mg par unité de prise et la quantité efficace sur le plan thérapeutique du mébendazole est de 100 mg par unité de prise.

6. Composition pharmaceutique apte à être administrée par voie orale selon la revendication 4, dans laquelle ladite composition pharmaceutique est destinée à une utilisation en pédiatrie et dans laquelle la quantité efficace sur le plan thérapeutique du nitazoxanide est de 100 mg par unité de prise et la quantité efficace sur le plan thérapeutique du mébendazole est de 50 mg par unité de prise.

7. Composition pharmaceutique apte à être administrée par voie orale selon la revendication 4, dans laquelle la composition pharmaceutique est en outre apte à être administrée toutes les 12 heures.

8. Composition pharmaceutique apte à être administrée par voie orale selon la revendication 4, dans laquelle la composition pharmaceutique est en outre apte à être administrée une fois par jour.

9. Composition pharmaceutique apte à être administrée par voie orale selon la revendication 8, dans laquelle la quantité efficace sur le plan thérapeutique du nitazoxanide est de 1000 mg et la quantité efficace sur le plan thérapeutique du mébendazole est de 200 mg par unité de prise.

10. Combinaison pharmaceutique comprenant une quantité efficace sur le plan thérapeutique de l'antiparasite nitazoxanide et une quantité efficace sur le plan thérapeutique de l'antiparasite mébendazole selon la revendication 1 pour le traitement de la parasitose chez l'homme.

11. Processus de préparation d'une composition pharmaceutique apte à être administrée par voie orale selon la revendication 1, comprenant la granulation, le mélange et la fabrication de comprimés d'une quantité efficace sur le plan thérapeutique de l'antiparasite nitazoxanide avec une quantité efficace sur le plan thérapeutique de l'antiparasite mébendazole, ainsi que des excipients acceptables sur le plan pharmaceutique.

12. Processus de préparation de la composition pharmaceutique apte à être administrée par voie orale selon la revendication 11, comprenant en outre le pelliculage de la composition pharmaceutique obtenue.

13. Processus de préparation d'une composition pharmaceutique à reconstitution extemporanée apte à être administrée par voie orale selon la revendication 3, comprenant le mélange d'une quantité efficace sur le plan thérapeutique de l'antiparasite nitazoxanide avec une quantité efficace sur le plan thérapeutique de l'antiparasite mébendazole, ainsi que des excipients acceptables sur le plan pharmaceutique.

14. Antiparasite nitazoxanide en combinaison avec un antiparasite mébendazole à utiliser dans le traitement de la parasitose chez l'homme.

15. Combinaison à utiliser selon la revendication 14, dans laquelle la parasitose chez l'homme est provoquée par les protozoaires et les helminthes.

**Fig. 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3950351 A
- US 5387598 A
- US 3657267 A

**Non-patent literature cited in the description**

- **SALOMÓN, M.C. et al.** *Parasitol. Latinoam,* June 2007, vol. 62 (1-2
- **SIRIVICHAYAKUL, C. et al.** *Southeast Asian J. Trop. Med. Public Health.,* 2001, vol. 32, 297-301
- **OLDS, G.R. et al.** *J. Infect. Diseases,* 1999, vol. 179, 996-1003
- **SOUKHATHAMMAVONG, PA.** *PLoS Negl Trop Dis.,* January 2012, vol. 6 (1), e1417
- **MILANO, A. et al.** Enteroparasitosis infantil intestinal en Argentina. *Medicina (Buenos Aires),* 2000, vol. 60, 23-4
- *J Antimicrob Chemother,* 2007, vol. 59 (2), 212-218